# EUROPEAN PATENT APPLICATION

(11) **EP 4 818 179 A1**
(43) Date of publication of application: **07.10.2026**
(21) Application number: 25747706.7
(22) Date of filing: 22.01.2025
(51) Int. Cl.: C07D 519/00, A61K 31/519, A61P 35/00, A61P 35/02

(54) **PREPARATION FOR AND USE OF PYRIMIDO-THIOPYRANDIONE INHIBITOR FOR KRAS G12C MUTANT PROTEIN**

(30) Priority: 01.02.2024 CN 202410145942
(71) Applicant: Ya Therapeutics Inc, Shanghai 201808 (CN)
(72) Inventor: LIANG, Yonghong, Shanghai 201808 (CN)
(74) Representative: Cleanthous, Marinos
(86) International application number: PCT/CN2025/074044
(87) International publication number: WO 2025/162135

(57) **Abstract**

The present invention relates to KRAS G12C mutant protein pyrimidothiopyranone inhibitors and their uses. Specifically, the present invention provides a compound shown in formula (I), where each substitution is defined as described in the specification. In addition, it also involves the combination of the inhibitor and its application. The compound of the present invention has good activity in inhibiting tumor growth, and it has good security.

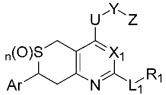

## Description

### CROSS-REFERENCE TO RELATED APPLICATIONS

This application claims priority to International Application Nos. PCT/CN 202410145942, filed on Feb 02, 2024, the content of each of which is incorporated herein by reference in its entirety.

### BACKGROUD OF THE INVENTION

RAS represents a population of 189 amino acid monomeric globular proteins (21 kDa molecular weight) that are associated with the plasma membrane and bind to GDP or GTP, and RAS acts as a molecular switch. When the RAS contains bound GDP, it is in a stationary or closed position and is "inactive." When cells are exposed to certain growth-promoting stimuli, RAS is induced to exchange their bound GDP for GTP. In the case of binding to GTP, RAS is "opened" and is capable of interacting with other proteins (its "downstream targets") and activating the proteins. The RAS protein itself has an inherently low ability to hydrolyze GTP back to GDP, thereby turning itself into a closed state. Closing RAS requires an exogenous protein called GTPase activating protein (GAP) that interacts with RAS and greatly accelerates the conversion of GTP to GDP. Any mutation in RAS that affects its ability to interact with GAP or convert GTP back to GDP will result in prolonged protein activation, and thus conduction to the cell to inform its signaling of continued growth and division. Since these signals cause cell growth and division, over-activated RAS signaling can ultimately lead to cancer.

Structurally, the RAS protein contains a G domain responsible for the enzymatic activity of RAS-guanine nucleotide binding and hydrolysis (GTPase reaction). It also contains a C-terminal extension called the CAAX cassette, which can be post-translationally modified and responsible for targeting the protein to the membrane. The G domain is approximately 21-25 kDa in size and contains a phosphate binding ring (P-ring). The P-loop represents a pocket of a binding nucleotide in a protein, and this is a rigid portion of a domain with conserved amino acid residues necessary for nucleotide binding and hydrolysis (glycine 12, threonine 26 and lysine 16). The G domain also contains a so-called switch I region (residues 30-40) and a switch II region (residues 60-76), both of which are dynamic parts of the protein, since the dynamic portion is converted between stationary and loaded states. The ability is often expressed as a "spring loaded" mechanism. The primary interaction is the hydrogen bond formed by threonine-35 and glycine-60 with the gamma-phosphate of GTP, which maintains the active conformation of the switch 1 region and the switch 2 region, respectively. After hydrolysis of GTP and release of phosphate, the two relax into an inactive GDP conformation.

The most notable members of the RAS subfamily are HRAS, KRAS and NRAS, which are primarily involved in many types of cancer. Mutation of any of the three major isoforms of the RAS gene (HRAS, NRAS or KRAS) is one of the most common events in human tumor formation. Approximately 30% of all tumors in human tumors were found to carry some mutations in the RAS gene. It is worth noting that KRAS mutations were detected in 25%-30% of tumors. In contrast, the rate of carcinogenic mutations in NRAS and HRAS family members was much lower (8% and 3%, respectively). The most common KRAS mutations were found at residues G12 and G13 in the P-loop as well as at residue Q61.G12C is a frequently occurring KRAS gene mutation (glycine-12 is mutated to cysteine). This mutation has been found in about 13% of cancers, about 43% in lung cancer, and almost 100% in MYH-associated polyposis (familial colon cancer syndrome).

As a frontier target, KRAS^{G12C} mutant protein has attracted extensive attention. Araxes (a subsidiary of Wellspring) developed the ARS-853 and ARS-1620 compounds in 2013 and 2016, respectively. In recent years, it has also filed a number of patents for KRAS^{G12C} inhibitors, such as WO2016164675 and WO2016168540. The ARS-853 compounds show good cell viability, but their pharmacokinetic properties are poor and are not suitable for evaluating the pharmacodynamics of animal models in vivo. ARS-1620 is highly efficient and selective against KRAS^{G12C}, enabling rapid and sustained target action in vivo to induce tumor regression. This study provides in vivo evidence that ARS-1620 represents a new generation of KRAS^{G12C}-specific inhibitors with significant therapeutic potential. Wellspring announced that the FDA has approved ARS-3248 for IND application. Other candidates for KRAS^{G12C} inhibitors include Mirati's MRTX-849 and Boehringer Ingelheim's Bi-2852.

Accordingly, while progress has been made in this field, there remains a need in the art for improved compounds and methods for treatment of cancer, for example by inhibition of KRAS, HRAS or NRAS. The present invention fulfills this need and provides further related advantages.

In brief, the present invention provides compounds, including stereoisomers, pharmaceutically acceptable salts, tautomers and prodrugs thereof which are capable of modulating G12C mutant KRAS, HRAS and/or NRAS proteins. In some instances, the compounds act as electrophiles which are capable of forming a covalent bond with the cysteine residue at position 12 of a KRAS, HRAS or NRAS G12C mutant protein. Methods for use of such compounds for treatment of various diseases or conditions, such as cancer, are also provided.

### Summary

The present invention includes compounds of Formula (I) and pharmaceutically acceptable salts or stereoisomer thereof as provided below and further defined herein: 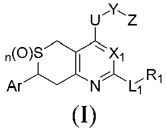 Wherein,
Each X₁ is selected from N, CR₄;
L₁ is selected from a single bond, -C₁₋₄alkyl-, -CR₈R₉-, -C₁₋₂alkyl(R⁸)(OH)-, -C(O)-, -CR₈R₉O-, -OCR₈R₉-, -SCR₈R₉-, -CR₈R₉S-, -NR₈-, -NR₈C(O)-, -C(O)NR₈-, -NR₈C(O)NR₉-, -CF₂-, -O-, -S-, -S(O)ₘ-, -NR₈S(O)ₘ-, -S(O)ₘNR₈-;
R₁ is selected from 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G¹;
R₄ is selected from H, D, CN, halogen, C₁₋₆ alkyl, COOH, NHCOH, CONH₂, OH or NH₂;
U is selected from a single bond, -C₁₋₄alkyl-, -CR₈R₉-, -C₁₋₂alkyl(R⁸)(OH)-, -C(O)-, -CR₈R₉O-, -OCR₈R₉-, -SCR₈R₉-, -CR₈R₉S-, -NR₈-, -NR₈C(O)-, -C(O)NR₈-, -NR₈C(O)NR₉-, -CF₂-, -O-, -S-, -S(O)ₘ-, -NR₈S(O)ₘ-, -S(O)ₘNR₈-;
Y is selected from a single bond, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G²;
Z is selected from CN, -NR₁₀CN, 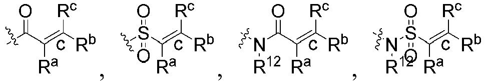
Bond c is selected from the double or triple bond;
Where c is the double bond, R^{a}, R^{b} and R^{c} are each independently selected from H, D, CN, halogen, C₁₋₆ alkyl, 3-6 membered cycloalkyl, 3-6 membered heterocycloalkyl. C₁₋₆ alkyl, 3-6 membered cycloalkyl, 3-6 membered heterocycloalkyl are optionally substituted with 1, 2 or 3 G³;
R^{a} and R^{b} or R^{b} and R^{c} are optionally connected to a carbon atom to form a 3-6-membered ring optionally containing heteroatoms;
Where c is the triple bond, R^{a} and R^{c}do not exist, while R^{b} is independently selected from H, deuterium, cyano, halogen, C1-6 alkyl, C3-6 cycloalkyl, or 3-6-membered heterocyclyl, C1-6 alkyl, C3-6 cycloalkyl, or 3-6-membered heterocyclyl are optionally substituted by one or more G⁴;
R₁₀ is independently selected from H, deuterium, cyano, halogen, C1-6 alkyl, C3-6 cycloalkyl, or 3-6-membered heterocyclyl, C1-6 alkyl, C3-6 cycloalkyl, or 3-6-membered heterocyclyl are optionally substituted by one or more G⁵;
Ar is independently selected from 5-12 membered heteroaromatic group or 6-10 membered aromatic group , 5-12 membered heteroaromatic group are optionally substituted by 1, 2, 3 or 4 heteroatom selected from N, O, or S, 5-12 membered heteroaromatic group are optionally substituted by one or more G⁶;
G¹, G², G³, G⁴, G⁵ and G⁶are each independently selected from deuterium, cyano, halogen, C1-6alkyl, C2-6alkenyl, C2-6alkynyl, C3-8cycloalkyl, or 3-8-membered heterocyclic, C6-10aryl, 5-10 membered heteroaromatic-, -OR₁₁, -OC(O)NR₁₁R₁₂, -C(O)OR₁₁, -C(O)NR₁₁R₁₂, -C(O)R₁₁, -NR₁₁R₁₂, -NR₁₁C(O)R₁₂, -NR₁₁C(O)NR₁₂R₁₃, -S(O)ₘR₁₁ or -NR₁₁S(O)ₘR₁₂, wherein the alkyl, vinyl, alkynyl, cyclic, heterocyclic, aromatic, and heteroaromatic groups are optionally substituted with one or more D, cyano, halogen, C1-6alkyl, C2-6alkenyl, C2-6alkynyl, C3-8alkyl, or 3-8-membered heterocyclic, C6-10aryl, 5-10 membered heteroaromatic groups, -OR₁₄, -OC(O)NR₁₄R₁₅, -C(O)OR₁₄, -C(O)NR₁₄R₁₅, -C(O)R₁₄, -NR₁₄R₁₅, -NR₁₄C(O)R₁₅, -NR₁₄C(O)NR₁₅R₁₆, -S(O)ₘR₁₄ or -NR₁₄S(O)ₙR₁₅;
R⁸, R⁹, R¹¹, R¹², R¹³, R¹⁴ and R¹⁵ are each independently selected from H, D, CN, halogen, C₁₋₆ alkyl, 3-8 membered cycloalkyl, 3-8 membered heterocycloalkyl, sing ring heteroaromatic or phenyl group;
m, n is selected from 1 or 2.

In some embodiments, the compound of formula (I), its pharmaceutically acceptable salt, or its stereoisomer, wherein formula (I) is further represented by (II-A), (II-B), (II-C), or (II-D)

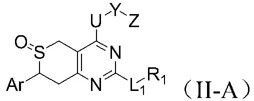

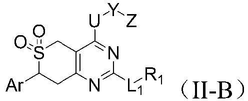

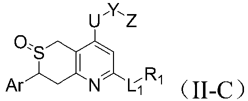

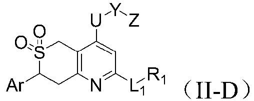

In some embodiments, the compound or its isomer, solvate, or precursor described in Formula (I), or its pharmaceutically acceptable salt, is selected from the following compounds, their isomers, solvates, or precursors, or their pharmaceutically acceptable salts.

In some embodiments of the present disclosure, the Ar is selected from:

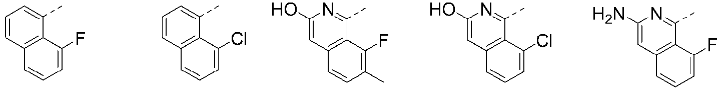

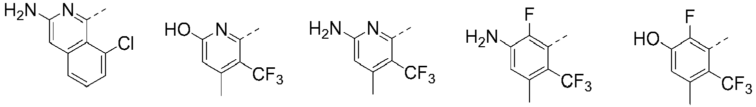

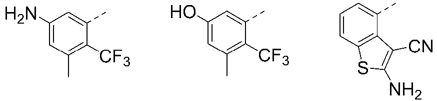

In some embodiments, the compound of formula (I) or an isomer, solvate or precursor thereof, or a pharmaceutically acceptable salt thereof is selected from the group consisting of the following compounds, isomers, solvates thereof or their precursors, or their pharmaceutically acceptable salts: 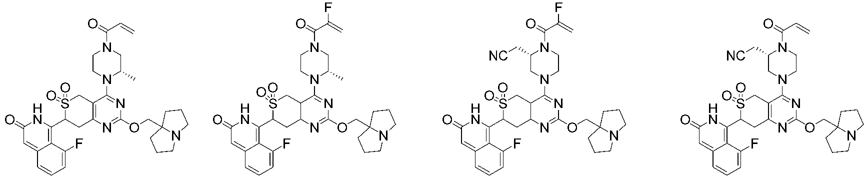 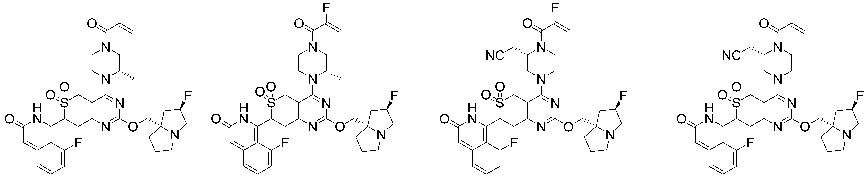 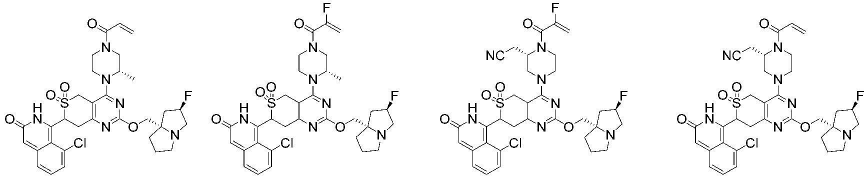 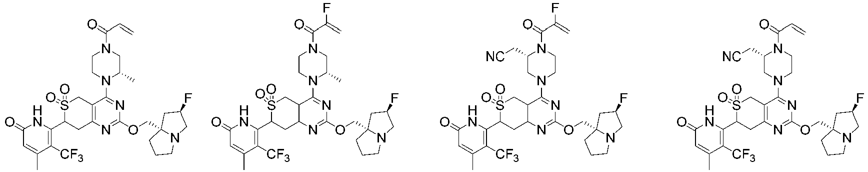 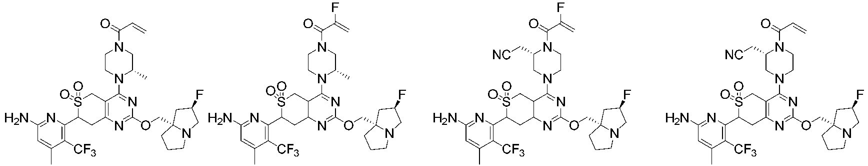 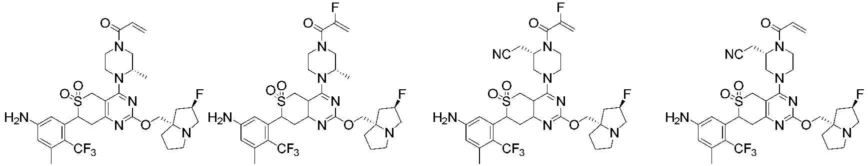 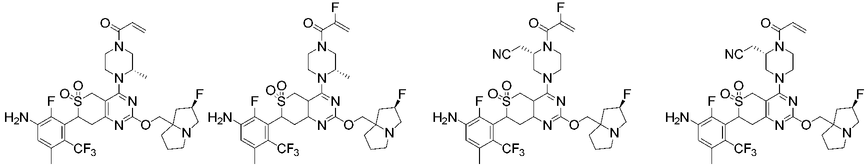 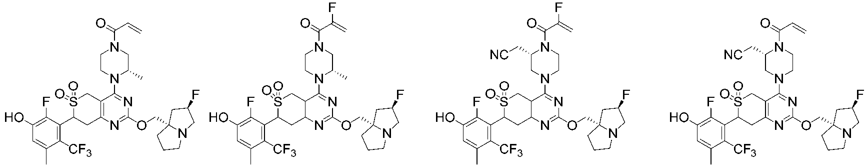 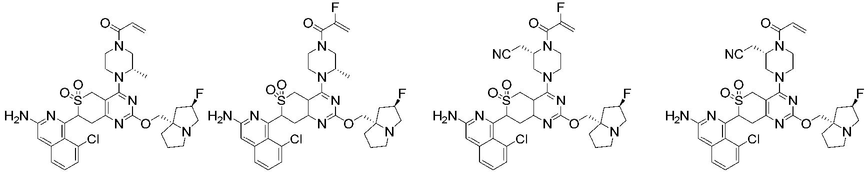 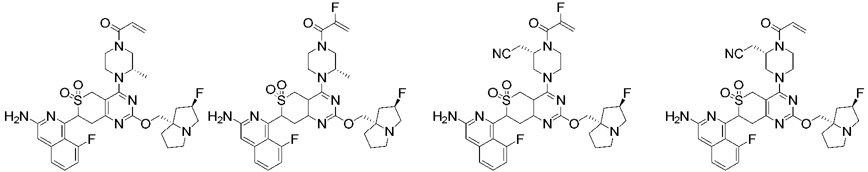 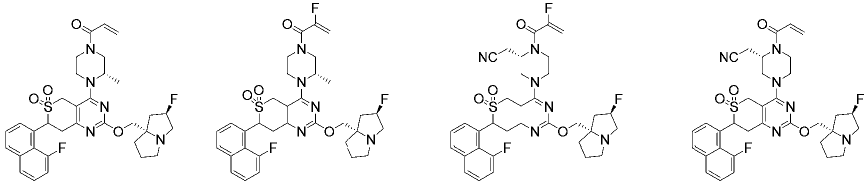 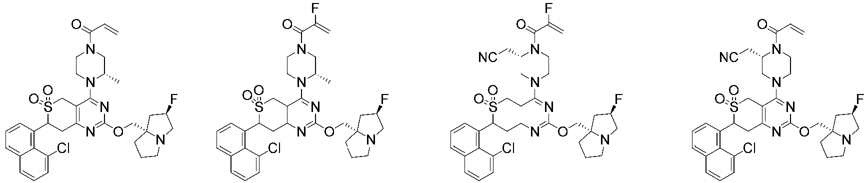 In some embodiments, the compound of formula (I) or an isomer, solvate or precursor thereof, or a pharmaceutically acceptable salt thereof is selected from the group consisting of the following compounds, isomers, solvates thereof or their precursors, or their pharmaceutically acceptable salts:

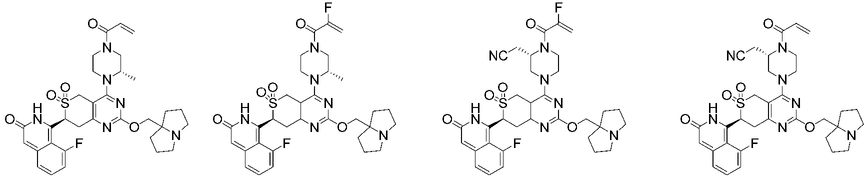

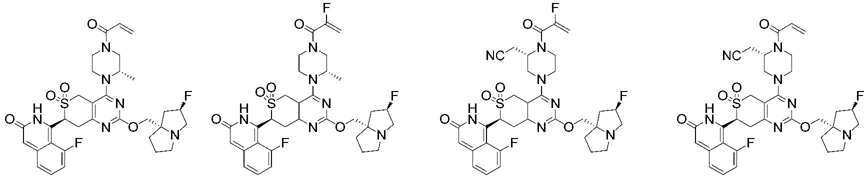

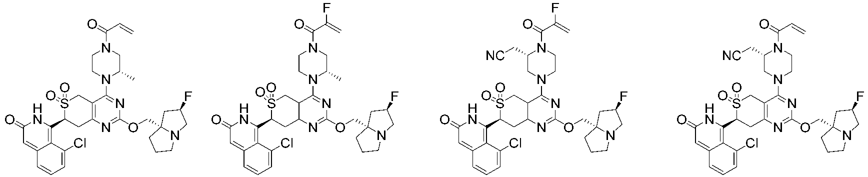

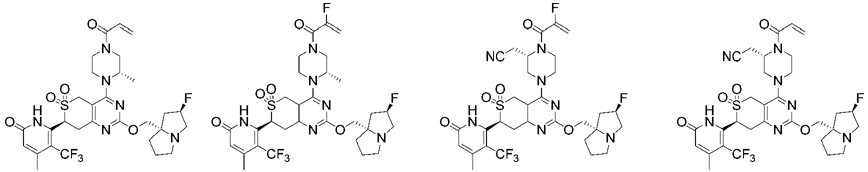

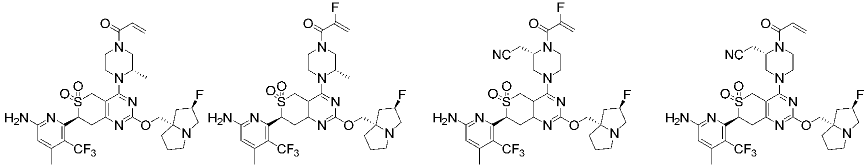

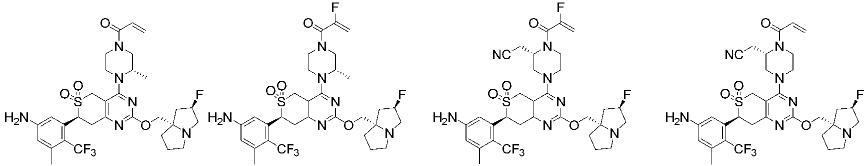

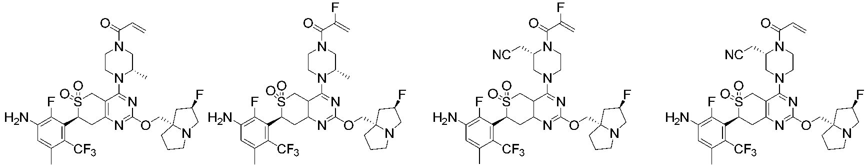

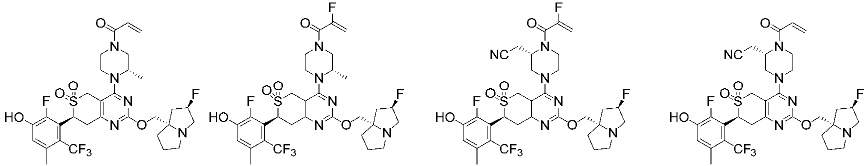

On the other hand, the present invention also provides a pharmaceutical composition comprising a compound of formula (I) or a pharmaceutically acceptable salt thereof, and a pharmaceutically acceptable excipient.

On the other hand, the present invention relates to a method for treating diseases associated with KRAS G12C in mammals, comprising administering a therapeutic effective amount of a compound of formula (I) or a pharmaceutically acceptable salt thereof, or a pharmaceutical combination thereof, to a mammal, preferably a human, in need of such treatment.

On the other hand, the present invention relates to the use of a compound represented by formula (I) or a pharmaceutically acceptable salt thereof for the prevention or treatment of KRAS G12C related diseases.

On the other hand, the present invention relates to a compound of formula (I) or a pharmaceutically acceptable salt thereof, or a pharmaceutical combination thereof, for the prevention or treatment of KRAS G12C related diseases.

### Specific implementation method

The present invention also provides methods for preparing the compounds. The preparation of the compounds of the general formula (I) of the present invention can be accomplished by the following exemplary methods and examples, but these methods and examples should not be construed as limiting the scope of the present invention in any way. The compounds of the present invention can also be synthesized using synthetic techniques known to those skilled in the art, or a combination of methods known in the art and methods described in the present invention. The products obtained in each step are obtained by separation techniques known in the art, including but not limited to extraction, filtration, distillation, crystallization, chromatographic separation, and the like. The starting materials and chemical reagents required for the synthesis can be routinely synthesized or purchased according to the literature (reaxys).

Unless otherwise stated, temperatures are in degrees Celsius. Reagents were purchased from commercial suppliers such as Sunwayor Maclean and these reagents were used without further purification unless otherwise stated.

Unless otherwise stated, the following reactions were performed at room temperature, in anhydrous solvent, under positive pressure of nitrogen or gas, or using a drying tube; glassware drying and/or heat drying.

Unless otherwise stated, column chromatography used 200-300 mesh silica gel from Qingdao Ocean Chemical Factory; preparative thin layer chromatography used thin layer chromatography silica gel prefabricated plate (HSGF254) produced by Yantai Chemical Industry Research Institute; MS was measured with Therno LCD Fleet type (ESI) liquid chromatography-mass spectrometer.
Nuclear magnetic data (¹H NMR) use Bruker Avance-400MHz or Varian Oxford-400Hz nuclear magnetic instrument, the solvents used in nuclear magnetic data are CDCl₃, CD₃OD, D₂O, DMSO-*d*₆, etc., based on tetramethylsilane (0.000ppm) or residual Solvent-based (CDCl₃: 7.26ppm; CD*₃*OD: 3.31ppm; D*₂*O: 4.79ppm; *d*₆-DMSO: 2.50ppm) When the peak shape diversity is indicated, the following abbreviations indicate different peak shapes: s (single peak), d (double peak) peak), t (triplet), q (quartet), m (multiplet), br (broad), dd (double doublet), dt (double triplet). If a coupling constant is given, it is in Hertz (Hz).

### Intermediate Synthesis

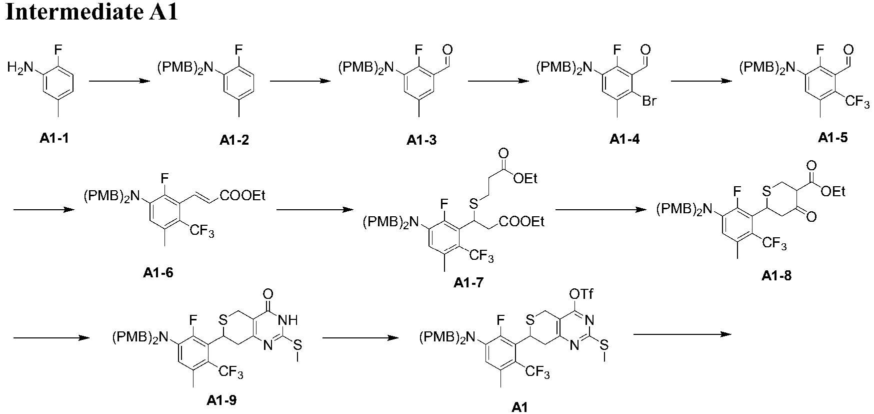

### Step A

In a dry 2L three necked bottle, NaH (0.8 g, 19 mmol) was added to DMF (10 mL), and the reaction system was heterogeneous gray. The temperature was lowered to 0 ° C, and a solution of compound **Al-1** (1 g, 8 mmol) in DMF (200 mL) was added dropwise under N₂ protection. The reaction was continued at 0 ° C for 0.5 hours, and para methoxybenzyl chloride (2.75 g, 17 mmol, 2.4 mL) was added. The temperature was slowly raised to 20 ° C, and stirring was continued for 7.5 hours under nitrogen protection. Slowly add the reaction solution to 10 mL of saturated ammonium chloride, extract with MTBE (10 mL × 2), wash the combined organic phases with 10 mL of saturated saline solution, dry with anhydrous Na₂SO₄, filter, and remove the organic solvent under reduced pressure. The crude product obtained is purified by silica gel column chromatography to obtain compound **Al-2.** LC-MS(ESI): m/z = 365.4[M+H]⁺.

### Step B

Add 2,2.6,6-tetramethylpiperidine (1.14 g, 8.21 mmol) to anhydrous THF (300 mL), cool to -5 ° C, add n-butyl lithium (2.5 M, 4 mL) dropwise, react at -5~0 ° C for 15 minutes, cool to -60 ° C, add compound **Al-2** (0.98 g, 2.7 mmol) in THF (3 mL) solution, react at -60 ° C for 0.5 hours, quickly add DMF (4 g, 0.05 mol), and continue stirring the reaction solution at -60 ° C for 10 minutes. Add 20 mL of saturated ammonium chloride to the reaction solution, extract with MTBE (10 mL × 2), wash the combined organic phases with 10 mL of saturated saline solution, dry with anhydrous Na₂SO₄, filter, and remove the organic solvent under reduced pressure. The crude product obtained is subjected to solvent pulping for 0.5 hours, filtered, dried from the filter cake, concentrated from the filtrate, and purified by silica gel column chromatography. Combine the filter cake and column chromatography to obtain compound A1-3.

LC-MS(ESI): m/z = 393.5[M+H]⁺.

### Step C

Add compound **A1-3** (0.98 g, 2.5 mmol) to 10 mL of DMF, and NBS (0.45 g, 2.5 mmol). Continue stirring the reaction mixture at 20 °C for 20 minutes. Add the reaction solution to 15 mL of water, extract with MTBE(8 mL × 2), wash the combined organic phases with saturated saline (5 mL × 2), dry with anhydrous Na₂SO₄, filter, and remove the organic solvent under reduced pressure. The crude product obtained is beaten with a mixed solvent for 0.5 hours, filtered, and the filter cake is dried to obtain compound **A1-4.** LC-MS(ESI): m/z = 472.4[M+H]⁺.

### Step D

Add compound **A1-4** (0.97 g, 2.04 mmol) to DMF (9.5 mL), and add cuprous iodide (0.78 g, 4.1 mmol) and methyl fluorosulfonyldifluoroacetate (2 g, 10 mmol) to the reaction solution under nitrogen. Heat the reaction solution to 100 ° C and continue stirring for 1 hour. Cool, filter the reaction solution through diatomaceous earth, add the filtrate to 15 mL of water, extract with MTBE (7.5 mL × 2), wash the combined organic phases with saturated saline (10 mL × 2), dry with anhydrous sodium sulfate, filter, and remove the organic solvent under reduced pressure. The crude product obtained is purified by silica gel column chromatography to obtain compound **A1-5.** LC-MS(ESI): m/z = 461.5[M+H]⁺.

### Step E

Add anhydrous THF (50 mL) and NaH (0.3 g, 7.3 mmol) to a dry three necked flask, cool to 0 °C, and add ethyl acetate (0.85 g, 7.3 mmol, 0.8 mL) dropwise under nitrogen protection. Continue stirring the reaction mixture at 0 ° C for 0.5 hours, and add n-butyl lithium (2.5 M, 3 mL) dropwise. Continue stirring the reaction mixture at this condition for 0.5 hours, cool to -60 ° C, and add compound **A1-5** (1.15 g, 2.5 mmol) in THF (5 mL) dropwise. Continue stirring the reaction mixture at -60 ° C for 0.5 hours. Add 25 mL of saturated ammonium chloride solution to the reaction solution, extract with 10 mL of ethyl acetate, wash the organic phase with 20 mL of saturated saline solution, dry with anhydrous sodium sulfate, filter, and remove the organic solvent under reduced pressure. The crude product obtained is purified by silica gel column chromatography to obtain compound **A1-6.** LC-MS(ESI): m/z =531.6[M+H]⁺.

### Step F

Add compound A1-6 (1 g, 1.9 mmol) to dichloromethane (10 mL), add 3-mercaptopropionic acid ethyl ester (0.3 g, 2.3 mmol) and titanium tetrachloride, and stir the reaction at room temperature for 48 hours. Filter, reduce pressure to remove organic solvents, and purify the crude product by silica gel column chromatography to obtain compound A1-7. LC/MS(ESI): m/z =665.7[M+H]⁺

### Step G

Compound **A1-7** (0.80 g, 1.2 mmol) and sodium ethoxide (0.57 mg, 1.9 mmol) were dissolved in 10 mL of THF and reacted with the solution at room temperature for 24 hours. The mixture was then filtered and dried to obtain **A1-8** LC/MS(ESI): m/z =619.7[M+H]⁺.

### Step H

To a suspension of **A1-8** (3.3 g, 5.3 mmol) in water (10 mL), S-methylisothiourea sulfate (1.7 g, 6.2 mmol) and potassium carbonate (1.6 g, 11.7 mmol) were added at room temperature. The reaction mixture was stirred for 20 hours. The precipitate was collected, washed twice with water and isopropyl ether, and dried to give **A1-9.** LC/MS(ESI): m/z = 645.7[M+H]⁺.

### Step I

Compound **A1-9** (1 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was pulped, filtered, and the filter cake was dried to obtain **A1**. LC/MS(ESI): m/z = 777.8[M+H]⁺.

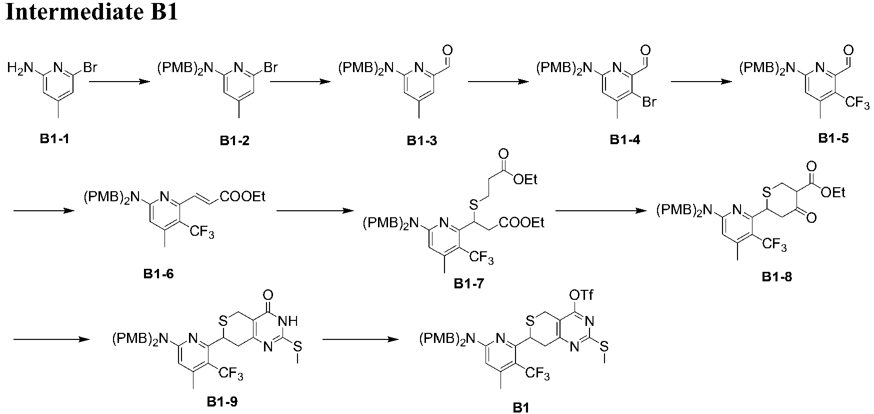

Compound **B1** was obtained using a similar preparation method to intermediate A1 (with the starting material replaced by 6-bromo-4-methylpyridine-2-amine). LC/MS(ESI): m/z =760.8[M+H]⁺.

### Example 1

### 1-((3S)-4-(7-(3-amino-2-fluoro-5-methyl-6-(trifluoromethyl)phenyl)-2-(((2R,7AS)-2-fluorotetrahydro-1H-pyrrolazin-7a(5H)-yl)methoxy)-6,6-dioxo-7,8-dihydro-5H-thiara n[4,3-d]pyrimidin-4-yl)-3-methylpyrazin-1-yl)prop-2-en-1-one (1)

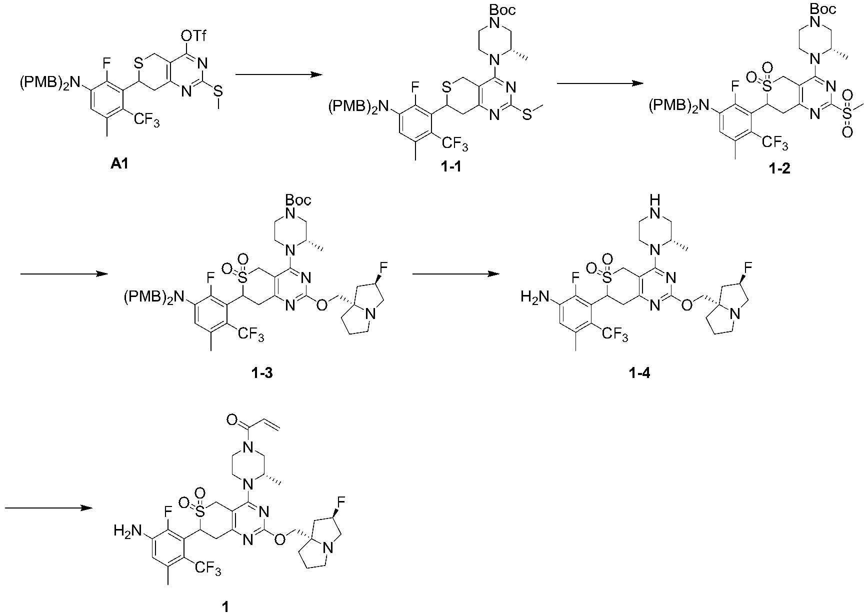

### Step A

Compound A1 (82 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain compound 1-1. LC/MS(ESI): m/z = 828[M+H]⁺.

### Step B

Compound **1-1** (71 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain compound **1-2.** LC/MS(ESI): m/z =892[M+H]⁺.

### Step C

Under ice-water bath conditions, compound ((2R,7AS)-2-fluorotetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound 1-2 (53 mg, 58.42 µmol) was added. The reaction mixture was stirred for 2 hours under ice-water bath conditions. The organic solvent was removed under reduced pressure, and the crude product was purified by thin-layer chromatography to obtain compound **1-3.** LC/MS(ESI): m/z =971[M+H]⁺.

### Step D

Compound **1-3** (42 mg, 42.78 µmol) were dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound 1-4. LC/MS(ESI): m/z =630.7[M+H]⁺.

### Step E

Compounds 1-4 (0.46 g, 0.73 mmol) and N,N-diisopropylethylamine (0.48 g, 3.75 mmol) were dissolved in dichloromethane (20 mL), and acryloyl chloride (59.73 mg, 0.66 mmol) was added at -78 °C, followed by stirring at -78 °C for 25 min. The reaction mixture was quenched with water and extracted with dichloromethane. The organic layers were mixed together. The organic layers were dried over anhydrous sodium sulfate and concentrated under vacuum. The residue was purified directly by reversed-phase chromatography to give compound **1.** LC-MS (ESI): m/z =684.7[M+H]⁺. ¹H-NMR (CD₃OD) *δ* 6.73-6.87 (m, 1H), 6.68 (d, 1H), 6.25 (dd, 1H), 5.46-5.58 (m, 1H), 4.58-4.74 (m, 5H), 3.94-4.43 (m, 8H), 3.24-3.79 (m, 4H), 2.89-3.13 (m, 2H), 2.03-2.32 (9 H), 1.31-1.42 (m, 3H).

### Example 2

### 1-((3S)-4-((7S)-7-(3-amino-2-fluoro-5-methyl-6-(trifluoromethyl)phenyl)-2-(((2R,7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-3-methylpyrazin-1-yl)prop-2-en-1-one (1A) 1-((3S)-4-((7R)-7-(3-amino-2-fluoro-5-methyl-6-(trifluoromethyl)phenyl)-2-(((2R,7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-3-methylpyrazin-1-yl)prop-2-en-1-one (1B)

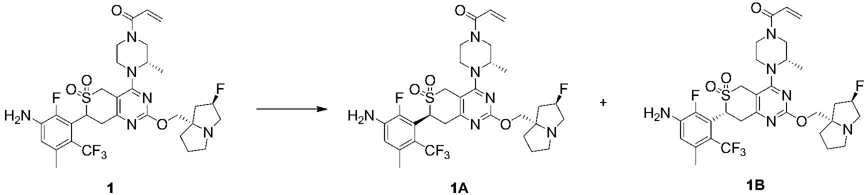

Compound 1 was separated and purified by high performance liquid chromatography (HPLC) to obtain 1A and 2A (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800; LC/MS (ESI): m/z =684.7[M+H]⁺.

### Example 3

### 1-((3S)-4-(7-(3-amino-2-fluoro-5-methyl-6-(trifluoromethyl)phenyl)-2-(((2R,7AS)-2-fluorotetrahydro-1H-pyrrolazin-7a(5H)-yl)methoxy)-6,6-dioxo-7,8-dihydro-5H-thiara n[4,3-d]pyrimidin-4-yl)-3-methylpyrazin-1-yl)-2-fluoroprop-2-en-1-one (2)

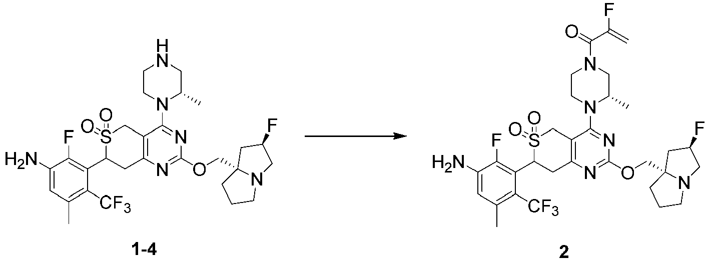

Compound **2** was obtained using a preparation method similar to step E in Example 1. LC/MS(ESI): m/z =702.7[M+H]⁺.

### Example 4

### 1-((3S)-4-((7S)-7-(3-amino-2-fluoro-5-methyl-6-(trifluoromethyl)phenyl)-2-(((2R,7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-3-methylpyrazin-1-yl)-2-fluoroprop-2-en-1-one (2A)

### 1-((3S)-4-((7R)-7-(3-amino-2-fluoro-5-methyl-6-(trifluoromethyl)phenyl)-2-(((2R,7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-3-methylpyrazin-1-yl)-2-fluoroprop-2-en-1-one (2B)

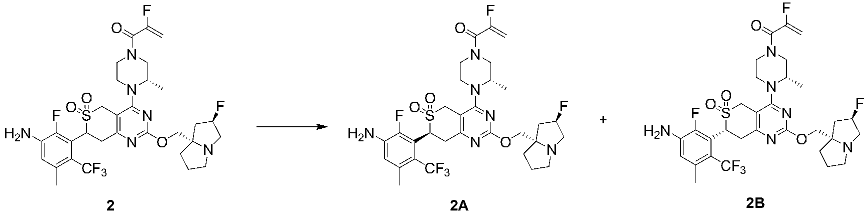

Compounds **2A** and **2B** were obtained using a similar preparation method to that used for compounds **1A** and **1B** in **Example 2** (with the raw materials replaced by compound 2). LC/MS(ESI): m/z =702.7[M+H]⁺.

### Example 5

### 2-((2S)-1-Acryloyl-4-(7-(3-amino-2-fluoro-5-methyl-6-(trifluoromethyl)phenyl)-2-((( 2R,7AS)-2-fluorotetrahydro-1H-pyrrolazin-7a(5H)-yl)methoxy)-6,6-dioxo-7,8-dihydr o-5H-thiaran[4,3-d]pyrimidin-4-yl)pyrazin-2-yl)acetonitrile (3)

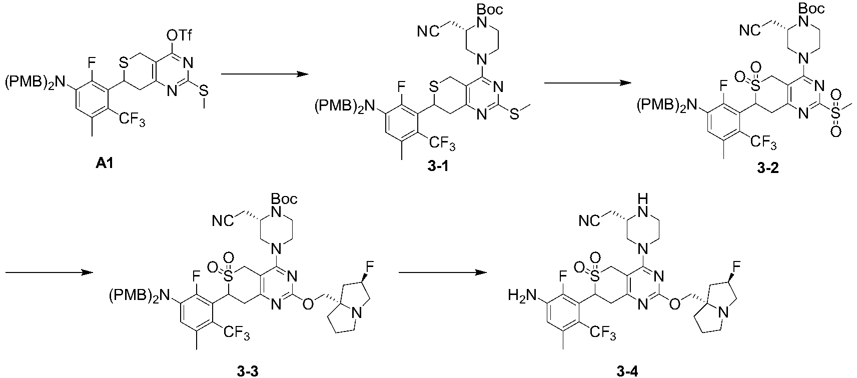

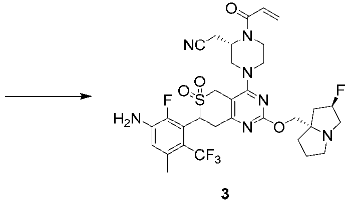

Compound **3** was prepared using a method similar to that used for compound **1** in **Example 1.** LC/MS(ESI): m/z =709.7[M+H]⁺. ¹H-NMR (CD₃OD) *δ* 6.73-6.87 (m, 1H), 6.68 (d, 1H), 6.25 (dd, 1H), 5.46-5.58 (m, 1H), 4.58-4.74 (m, 5H), 3.94-4.43 (m, 8H), 3.15-3.79 (m, 6H), 2.89-3.13 (m, 2H), 2.16-2.82 (m, 9H).

### Example 6

### 2-((2S)-1-Acryloyl-4-((7S)-7-(3-amino-2-fluoro-5-methyl-6-(trifluoromethyl)phenyl)-2-(((2R,7AS)-2-fluorotetrahydro-1H-pyrrolazin-7a(5H)-yl)methoxy)-6,6-dioxo-7,8-di hydro-5H-thiaran[4,3-d]pyrimidin-4-yl)pyrazin-2-yl)acetonitrile (3A)

### 2-((2S)-1-Acryloyl-4-((7R)-7-(3-amino-2-fluoro-5-methyl-6-(trifluoromethyl)phenyl) -2-(((2R,7AS)-2-fluorotetrahydro-1H-pyrrolazin-7a(5H)-yl)methoxy)-6,6-dioxo-7,8-d ihydro-5H-thiaran[4,3-d]pyrimidin-4-yl)pyrazin-2-yl)acetonitrile (3B)

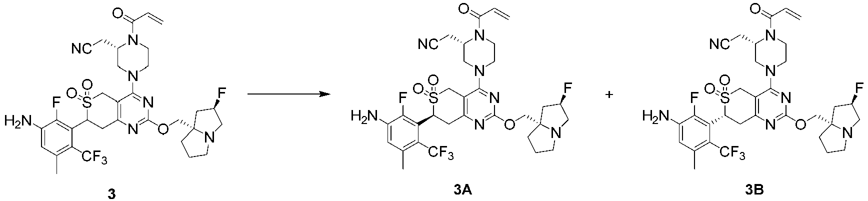

Compounds **3A** and **3B** were obtained using a similar preparation method to that used for compounds **1A** and **1B** in **Example 2** (with the raw materials replaced by compound 2). LC/MS(ESI): m/z =709.7[M+H]⁺.

### Example 7

### 2-((2S)-4-(7-(3-amino-2-fluoro-5-methyl-6-(trifluoromethyl)phenyl)-2-(((2R,7AS)-2-fluorotetrahydro-1H-pyrrolazin-7a(5H)-yl)methoxy)-6,6-dioxo-7,8-dihydro-5H-thiara n[4,3-d]pyrimidin-4-yl)-1-(2-fluoroacryloyl)pyrazin-2-yl)acetonitrile (4)

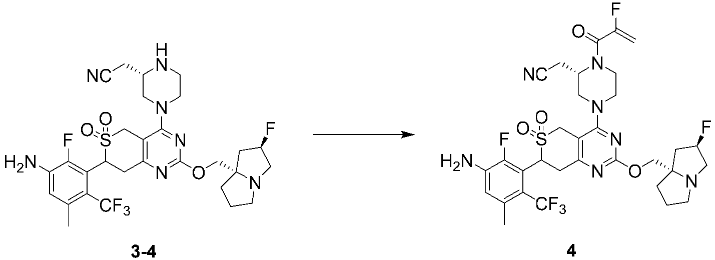

Compound **4** was obtained using a preparation method similar to step **E** in **Example 1.** LC/MS(ESI): m/z =727.7[M+H]⁺. ¹H-NMR (CD₃OD) *δ* 6.72-6.84 (m, 1H), 5.46-5.58 (m, 1H), 5.19-5.44 (m, 1H), 4.48-4.79 (m, 5H), 3.94-4.43 (m, 8H), 3.16-3.79 (m, 6H), 2.89-3.13 (m, 2H), 2.18-2.82 (m, 9H).

### Example 8

### 2-((2S)-4-((7S)-7-(3-amino-2-fluoro-5-methyl-6-(trifluoromethyl)phenyl)-2-(((2R, 7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-1-(2-fluoroacryloyl)pyrazin-2-yl)acetonitrile (4A)

### 2-((2S)-4-((7R)-7-(3-amino-2-fluoro-5-methyl-6-(trifluoromethyl)phenyl)-2-(((2R, 7A S)-2-fluorotetrahydro-1H-pyrrolazin-7a(5H)-yl)methoxy)-6,6-dioxo-7,8-dihydro-5H-t hiaran[4,3-d]pyrimidin-4-yl)-1-(2-fluoroacryloyl)pyrazin-2-yl)acetonitrile (4B)

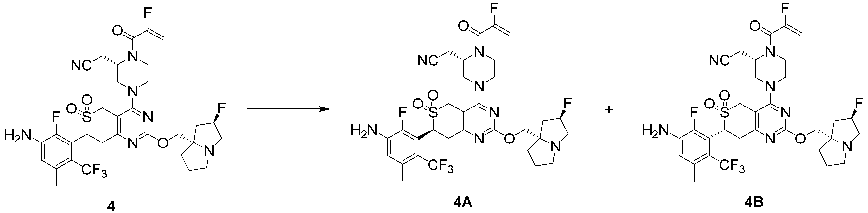

Compounds **4A** and **4B** were obtained using a similar preparation method to that used for compounds **1A** and 1**B** in **Example 2** (with the raw material replaced by compound **4).** LC/MS(ESI): m/z =727.7[M+H]⁺.

### Example 9

### 1-((3S)-4-(7-(6-amino-4-methyl-3-(trifluoromethyl)pyridin-2-yl)-2-(((2R,7AS)-2-fluo rotetrahydro-1H-pyrrolazin-7a(5H)-yl)methoxy)-6,6-dioxo-7,8-dihydro-5H-thiaran[4, 3-d]pyrimidin-4-yl)-3-methylpyrazin-1-yl)prop-2-en-1-one (5)

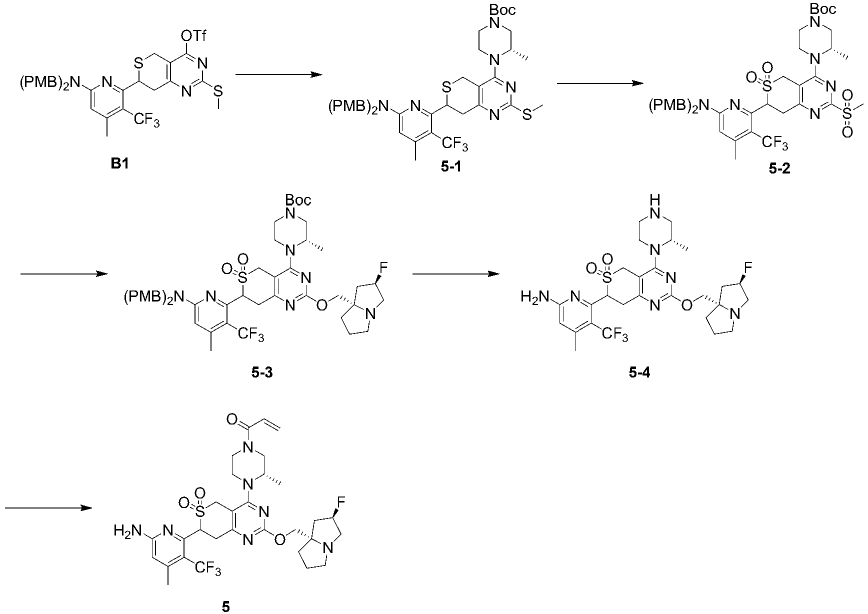

Compound **5** was prepared using a similar method to that used for compound 1 in **Example 1** (with the starting material replaced by **B1**). LC/MS(ESI): m/z =667.7[M+H]⁺.

### Example 10

### 1-((3S)-4-((7S)-7-(6-amino-4-methyl-3-(trifluoromethyl)pyridin-2-yl)-2-(((2R,7AS)-2 -fluorotetrahydro-1H-pyrrolazin-7a(5H)-yl)methoxy)-6,6-dioxo-7,8-dihydro-5H-thiar an[4,3-d]pyrimidin-4-yl)-3-methylpyrazin-1-yl)prop-2-en-1-one (5A)

### 1-((3S)-4-((7R)-7-(6-amino-4-methyl-3-(trifluoromethyl)pyridin-2-yl)-2-(((2R,7AS)-2-fluorotetrahydro-1H-pyrrolazin-7a(5H)-yl)methoxy)-6,6-dioxo-7,8-dihydro-5H-thia ran[4,3-d]pyrimidin-4-yl)-3-methylpyrazin-1-yl)prop-2-en-1-one (5B)

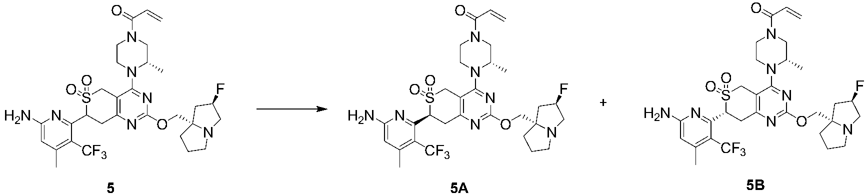

Compounds **5A** and **5B** were obtained using a similar preparation method to that used for compounds **1A** and **1B** in **Example 2** (with compound **5** as the starting material). LC/MS(ESI): m/z =667.7[M+H]⁺.

### Example 11

### 1-((3S)-4-(7-(6-amino-4-methyl-3-(trifluoromethyl)pyridin-2-yl)-2-(((2R,7AS)-2-fluo rotetrahydro-1H-pyrrolazin-7a(5H)-yl)methoxy)-6,6-dioxo-7,8-dihydro-5H-thiaran[4, 3-d]pyrimidin-4-yl)-3-methylpyrazin-1-yl)-2-fluoroprop-2-en-1-one (6)

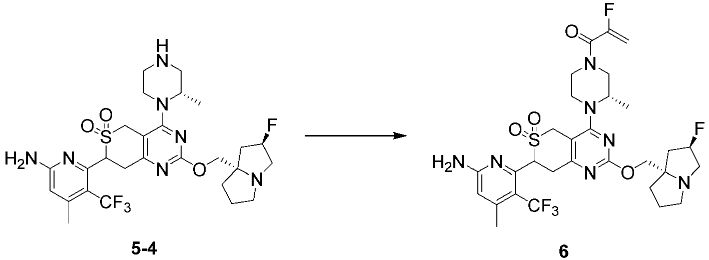

Compound **6** was obtained using a preparation method similar to **step E** in **Example 1.** LC/MS(ESI): m/z =685.7[M+H]⁺.

### Example 12

### 1-((3S)-4-((7S)-7-(6-amino-4-methyl-3-(trifluoromethyl)pyridin-2-yl)-2-(((2R,7AS)-2 -fluorotetrahydro-1H-pyrrolazin-7a(5H)-yl)methoxy)-6,6-dioxo-7,8-dihydro-5H-thiar an[4,3-d]pyrimidin-4-yl)-3-methylpyrazin-1-yl)-2-fluoroprop-2-en-1-one (6A)

### 1-((3S)-4-((7R)-7-(6-amino-4-methyl-3-(trifluoromethyl)pyridin-2-yl)-2-(((2R,7AS)-2-fluorotetrahydro-1H-pyrrolazin-7a(5H)-yl)methoxy)-6,6-dioxo-7,8-dihydro-5H-thia ran[4,3-d]pyrimidin-4-yl)-3-methylpyrazin-1-yl)-2-fluoroprop-2-en-1-one(6B)

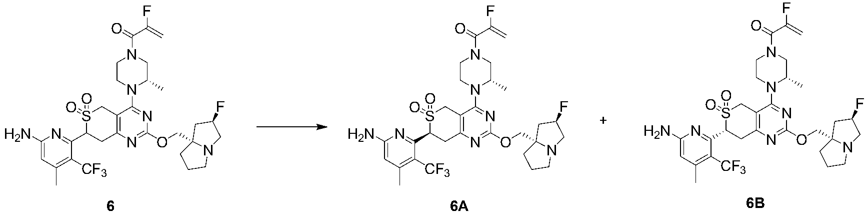

Compounds **6A** and **6B** were obtained using a similar preparation method to that used for compounds **1A** and **1B** in **Example 2** (with the raw material replaced by compound **6).** LC/MS(ESI): m/z =685.7[M+H]⁺.

### Example 13

### 2-((2S)-4-(7-(6-amino-4-methyl-3-(trifluoromethyl)pyridin-2-yl)-2-(((2R,7AS)-2-fluo rotetrahydro-1H-pyrrolazin-7a(5H)-yl)methoxy)-6,6-dioxo-7,8-dihydro-5H-thiaran[4, 3-d]pyrimidin-4-yl)-1-(acryloyl)pyrazin-2-yl)acetonitrile (7)

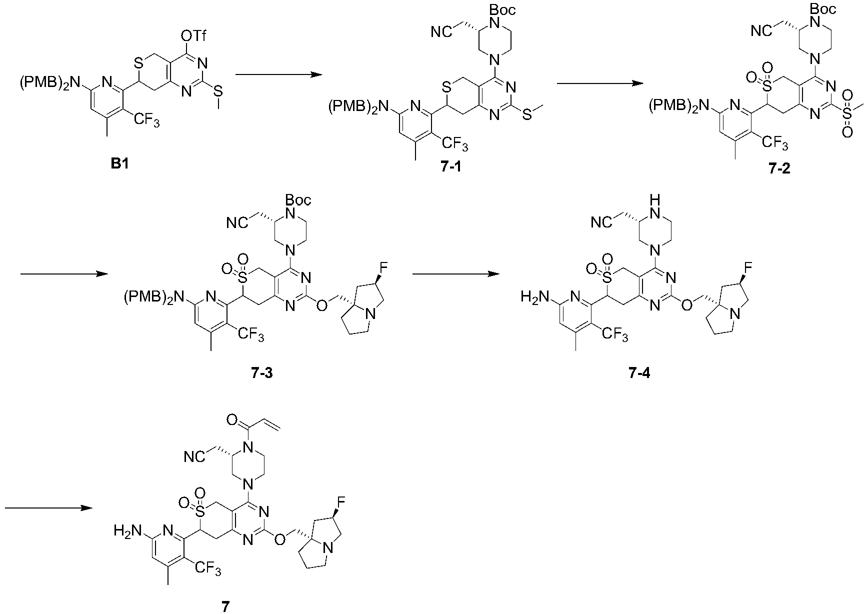

Compound **7** was obtained using a similar preparation method to that of Compound **1** in **Example 1** (with the starting material replaced by **Bl).** LC/MS(ESI): m/z =692.7[M+H]⁺.

### Example 14

### 2-((2S)-4-((7S)-7-(6-amino-4-methyl-3-(trifluoromethyl)pyridin-2-yl)-2-(((2R,7AS)-2 -fluorotetrahydro-1H-pyrrolazin-7a(5H)-yl)methoxy)-6,6-dioxo-7,8-dihydro-5H-thiar an[4,3-d]pyrimidin-4-yl)-1-(acryloyl)pyrazin-2-yl)acetonitrile (7A)

### 2-((2S)-4-((7R)-7-(6-amino-4-methyl-3-(trifluoromethyl)pyridin-2-yl)-2-(((2R,7AS)-2-fluorotetrahydro-1H-pyrrolazin-7a(5H)-yl)methoxy)-6,6-dioxo-7,8-dihydro-5H-thia ran[4,3-d]pyrimidin-4-yl)-1-(acryloyl)pyrazin-2-yl)acetonitrile (7B)

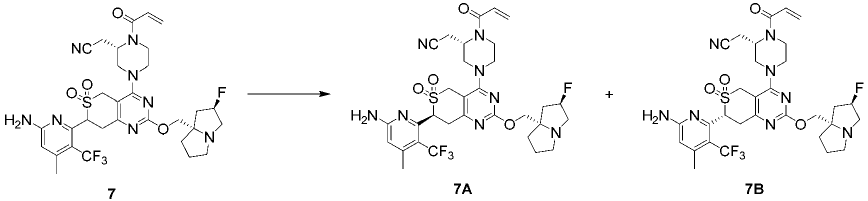

Compounds **7A** and **7B** were obtained using a similar preparation method to that used for compounds **1A** and **1B** in **Example 2** (with the raw material replaced by compound **7).** LC/MS(ESI): m/z =692.7[M+H]⁺.

### Example 15

### 2-((2S)-4-(7-(6-amino-4-methyl-3-(trifluoromethyl)pyridin-2-yl)-2-(((2R,7AS)-2-fluo rotetrahydro-1H-pyrrolazin-7a(5H)-yl)methoxy)-6,6-dioxo-7,8-dihydro-5H-thiaran[4, 3-d]pyrimidin-4-yl)-1-(2-fluoroacryloyl)pyrazin-2-yl)acetonitrile (8)

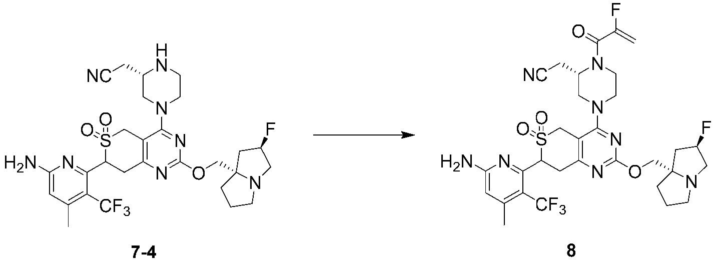

Compound **8** was obtained using a preparation method similar to **step E** in **Example 1.** LC/MS(ESI): m/z =710.7[M+H]⁺.

### Example 16

### 2-((2S)-4-((7S)-7-(6-amino-4-methyl-3-(trifluoromethyl)pyridin-2-yl)-2-(((2R,7AS)-2 -fluorotetrahydro-1H-pyrrolazin-7a(5H)-yl)methoxy)-6,6-dioxo-7,8-dihydro-5H-thiar an[4,3-d]pyrimidin-4-yl)-1-(2-fluoroacryloyl)pyrazin-2-yl)acetonitrile (8A)

### 2-((2S)-4-((7R)-7-(6-amino-4-methyl-3-(trifluoromethyl)pyridin-2-yl)-2-(((2R,7AS)-2-fluorotetrahydro-1H-pyrrolazin-7a(5H)-yl)methoxy)-6,6-dioxo-7,8-dihydro-5H-thia ran[4,3-d]pyrimidin-4-yl)-1-(2-fluoroacryloyl)pyrazin-2-yl)acetonitrile (8B)

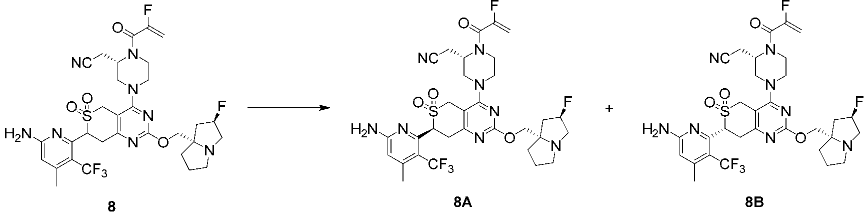

Compounds **8A** and **8B** were obtained using a similar preparation method to that used for compounds 1A and 1B in Example 2 (with the raw material replaced by compound 8). LC/MS(ESI): m/z =710.7[M+H]⁺.

### Example 17 Bioactivity Test

The present invention is further described and explained below with reference to test examples, but these embodiments are not intended to limit the scope of the invention.

### I. Tumor Cell Proliferation Inhibition Experiment

### 1. Experimental Method

H358 (KRAS G12C mutant) cells were digested, centrifuged, and resuspended. Cell density was determined using a Scepter automated cell counter. The cells were diluted to a solution containing 44,000 cells per milliliter. The adjusted cell solution was added to 90 µL per well in a 96-well culture plate. The 96-well plate was placed in a 37°C, 5% CO₂ incubator. After culturing the cells for 24 hours, different concentrations of the test compound were added. The cells were then cultured with the compound for 72 hours in the presence of 10% fetal bovine serum. The ATP content was measured using the Cell Titer-Glo luminescent cell viability assay kit (see manufacturer's instructions for details) to assess cell growth inhibition. In short, 30 µL of Cell Titer-Glo reagent was added to each well, and the plate was shaken for 10 minutes to induce cell lysis. The fluorescence signal was detected and recorded using a Fluoroskan Ascent FL (Thermo). The maximum signal value was obtained from cells treated with dimethyl sulfoxide for 72 hours. Minimum signal value was obtained from a single culture medium (zero cell count). Inhibition rate % = (maximum signal value - minimum signal value) / (maximum signal value - minimum signal value × 100%). Data was processed using GraphPadprism 5 software. IC50 values were calculated by fitting an S-shaped dose-response curve. "A" indicates IC₅₀ ≤ 10 nM; "B" indicates 10 < IC₅₀ ≤ 100 nM; "C" indicates 100 < IC₅₀ ≤ 1000 nM; "D" indicates 1000 nM < IC₅₀.

2. Experimental Results The IC₅₀ values of each compound in the above experiments were calculated, and the results are shown in Table 1 below.

**Table 1. Inhibitory activity of compounds against tumor cell proliferation (IC₅₀, nm).**

| NO | NCI-H538 IC₅₀(nm) | NO | NCI-H538 IC₅₀(nm) | NO | NCI-H538 IC₅₀(nm) |
|---|---|---|---|---|---|
| 1 | A | 4 | A | 7 | A |
| 1A | A | 4A | A | 7A | A |
| 1B | A | 4B | A | 7B | A |
| 2 | A | 5 | A | 8 | A |
| 2A | A | 5A | A | 8A | A |
| 2B | A | 5B | A | 8B | A |
| 3 | A | 6 | A | | |
| 3A | A | 6A | A | | |
| 3B | A | 6B | A | | |

In vitro kinase activity tests showed that the designed compounds exhibited strong inhibitory activity against the KRASG12C mutant strain.

Although the present invention has been described in detail above, those skilled in the art will understand that various modifications and changes can be made to the invention without departing from its spirit and scope. The scope of the invention is not limited to the detailed description above, but should be attributed to the claims.

## Claims

1. A compound having the general formula (I), its stereoisomers, pharmaceutically acceptable salts, polymorphs, or isomers, wherein the compound having the general formula (I) has the following structure: 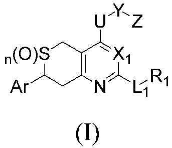 Wherein,
Each X₁ is selected from N, CR₄;
L₁ is selected from a single bond, -C₁₋₄alkyl-, -CR₈R₉-, -C₁₋₂alkyl(R⁸)(OH)-, -C(O)-, -CR₈R₉O-, -OCR₈R₉-, -SCR₈R₉-, -CR₈R₉S-, -NR₈-, -NR₈C(O)-, -C(O)NR₈-, -NR₈C(O)NR₉-, -CF₂-, -O-, -S-, -S(O)ₘ-, -NR₈S(O)ₘ-, -S(O)ₘNR₈-;
R₁ is selected from 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G¹;
R₄ is selected from H, D, CN, halogen, C₁₋₆ alkyl, COOH, NHCOH, CONH₂, OH or NH₂;
U is selected from a single bond, -C₁₋₄alkyl-, -CR₈R₉-, -C₁₋₂alkyl(R⁸)(OH)-, -C(O)-, -CR₈R₉O-, -OCR₈R₉-, -SCR₈R₉-, -CR₈R₉S-, -NR₈-, -NR₈C(O)-, -C(O)NR₈-, -NR₈C(O)NR₉-, -CF₂-, -O-, -S-, -S(O)ₘ-, -NR₈S(O)ₘ-, -S(O)ₘNR₈-;
Y is selected from a single bond, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G²;
Z is selected from CN, -NR₁₀CN, 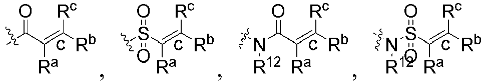
Bond c is selected from the double or triple bond;
Where c is the double bond, R^{a}, R^{b} and R^{c} are each independently selected from H, D, CN, halogen, C₁₋₆ alkyl, 3-6 membered cycloalkyl, 3-6 membered heterocycloalkyl. C₁₋₆ alkyl, 3-6 membered cycloalkyl, 3-6 membered heterocycloalkyl are optionally substituted with 1, 2 or 3 G³;
R^{a} and R^{b} or R^{b} and R^{c} are optionally connected to a carbon atom to form a 3-6-membered ring optionally containing heteroatoms;
Where c is the triple bond, R^{a} and R^{c}do not exist, while R^{b} is independently selected from H, deuterium, cyano, halogen, C1-6 alkyl, C3-6 cycloalkyl, or 3-6-membered heterocyclyl, C1-6 alkyl, C3-6 cycloalkyl, or 3-6-membered heterocyclyl are optionally substituted by one or more G⁴;
R₁₀ is independently selected from H, deuterium, cyano, halogen, C1-6 alkyl, C3-6 cycloalkyl, or 3-6-membered heterocyclyl, C1-6 alkyl, C3-6 cycloalkyl, or 3-6-membered heterocyclyl are optionally substituted by one or more G⁵;
Ar is independently selected from 5-12 membered heteroaromatic group or 6-10 membered aromatic group , 5-12 membered heteroaromatic group are optionally substituted by 1, 2, 3 or 4 heteroatom selected from N, O, or S, 5-12 membered heteroaromatic group are optionally substituted by one or more G⁶;
G¹, G², G³, G⁴, G⁵ and G⁶are each independently selected from deuterium, cyano, halogen, C1-6alkyl, C2-6alkenyl, C2-6alkynyl, C3-8cycloalkyl, or 3-8-membered heterocyclic, C6-10aryl, 5-10 membered heteroaromatic-, -OR₁₁, -OC(O)NR₁₁R₁₂, -C(O)OR₁₁, -C(O)NR₁₁R₁₂, -C(O)R₁₁, -NR₁₁R₁₂, -NR₁₁C(O)R₁₂, -NR₁₁C(O)NR₁₂R₁₃, -S(O)ₘR₁₁ or -NR₁₁S(O)ₘR₁₂, wherein the alkyl, vinyl, alkynyl, cyclic, heterocyclic, aromatic, and heteroaromatic groups are optionally substituted with one or more D, cyano, halogen, C1-6alkyl, C2-6alkenyl, C2-6alkynyl, C3-8alkyl, or 3-8-membered heterocyclic, C6-10aryl, 5-10 membered heteroaromatic groups-, -OR₁₄, -OC(O)NR₁₄R₁₅, -C(O)OR₁₄, -C(O)NR₁₄R₁₅, -C(O)R₁₄, -NR₁₄R₁₅, -NR₁₄C(O)R₁₅, -NR₁₄C(O)NR₁₅R₁₆, -S(O)ₘR₁₄ or -NR₁₄S(O)ₙR₁₅;
R⁸, R⁹, R¹¹, R¹², R¹³, R¹⁴ and R¹⁵ are each independently selected from H, D, CN, halogen, C₁₋₆ alkyl, 3-8 membered cycloalkyl, 3-8 membered heterocycloalkyl, sing ring heteroaromatic or phenyl group;
m, n is selected from 1 or 2.

2. The compound of claim 1 or a pharmaceutically acceptable salt thereof, wherein the compound is represented by the general formula (II-A), (II-B), (II-C) or (II-D):

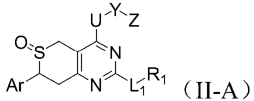

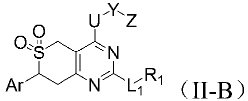

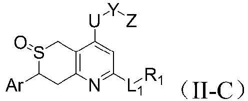

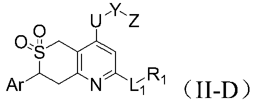

3. The compound according to claim 1 or a pharmaceutically acceptable salt thereof, wherein Ar is selected from:

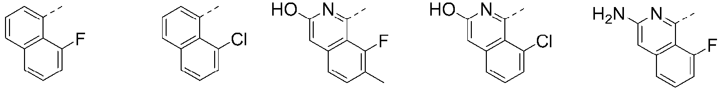

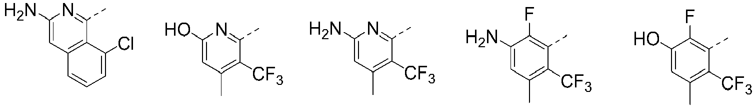

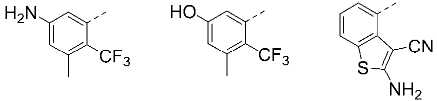

4. The compound according to claim 1, or its prodrug, stable isotope derivative, pharmaceutically acceptable salt, solvate, polymorph or isomer, or mixture thereof, selected from the following compounds:

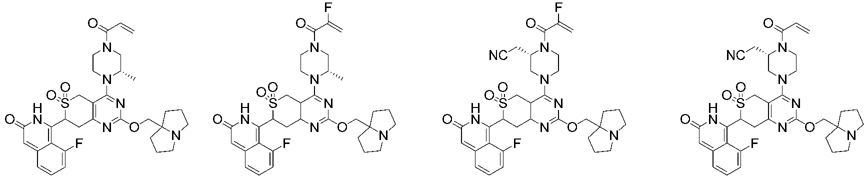

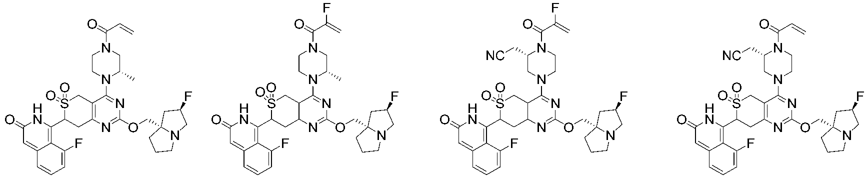

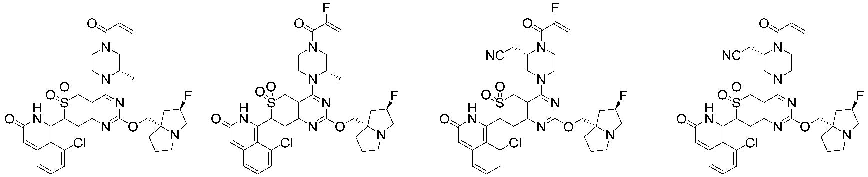

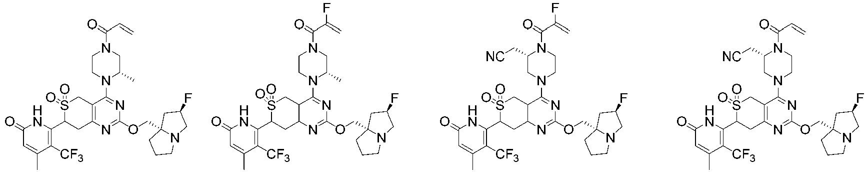

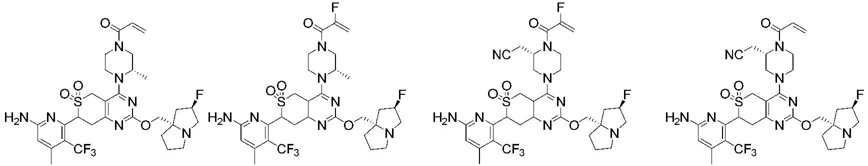

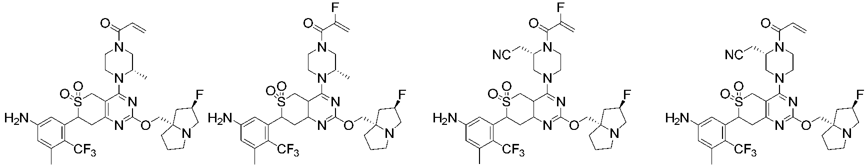

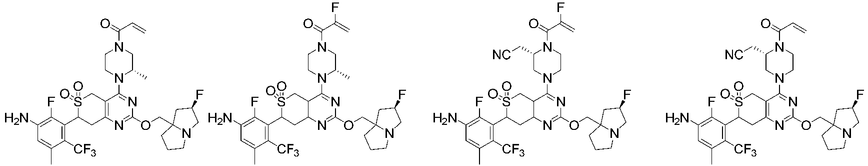

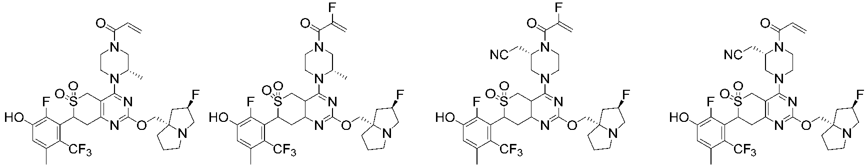

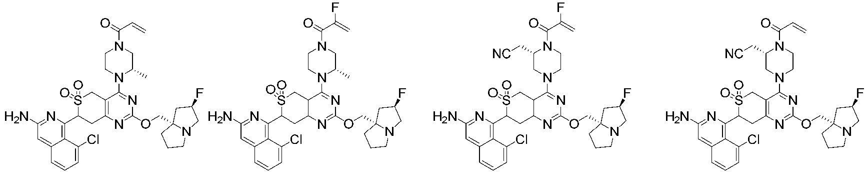

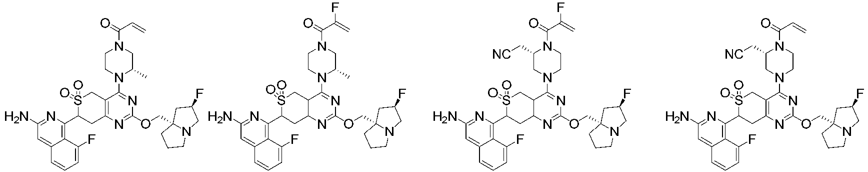

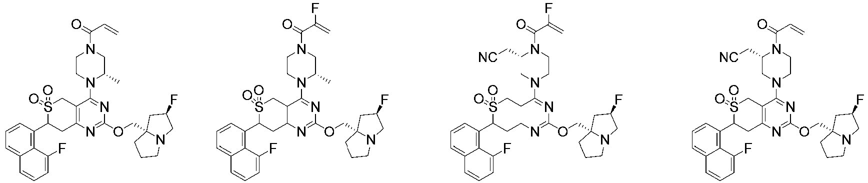

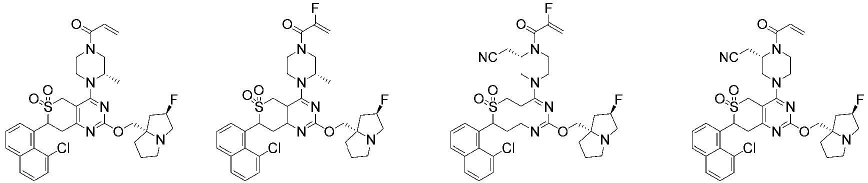

5. The compound according to any one of claims 4, or a pharmaceutically acceptable salt thereof, selected from:

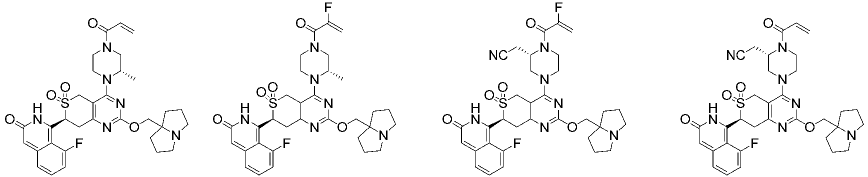

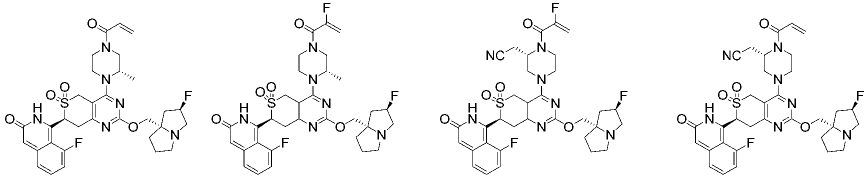

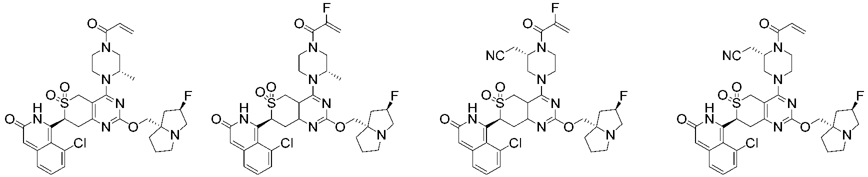

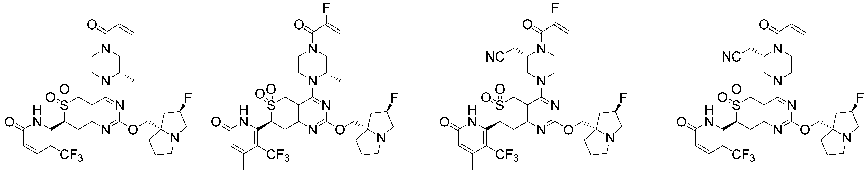

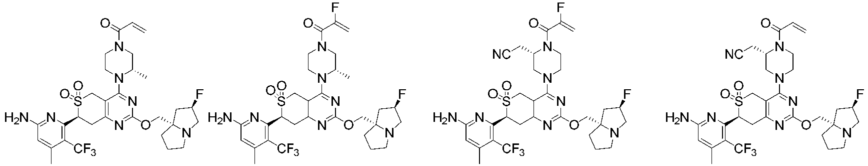

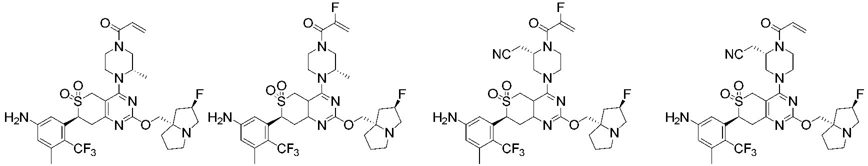

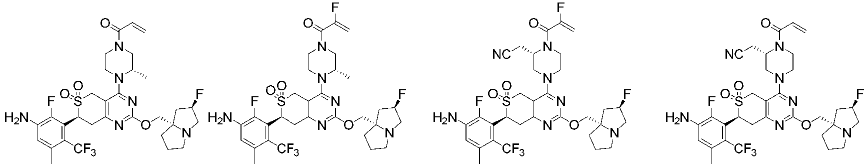

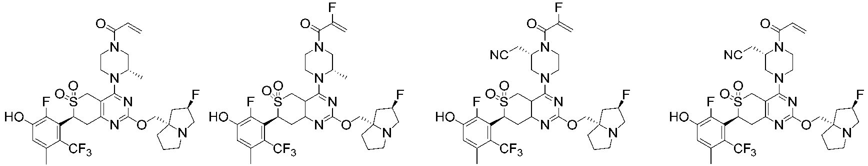

6. The compound of any one of claims 1-5, or an optical isomer thereof, a pharmaceutically acceptable salt, a prodrug, a deuterated derivative, a hydrate, or a solvate, **characterized in that** the pharmaceutically acceptable salt is selected from the group consisting of potassium, sodium, magnesium, calcium, sulfate, hydrochloride, phosphate, sulfonate, or carbonate.

7. A pharmaceutical composition, **characterized in that** it comprises the compound of any one of claims 1-6, or an optical isomer thereof, a pharmaceutically acceptable salt, a prodrug, a deuterated derivative, a hydrate, a solvate, and a pharmaceutically acceptable carrier.

8. Use of the compound of any one of claims 1-6, or an optical isomer thereof, a pharmaceutically acceptable salt, a prodrug, a deuterated derivative, a hydrate, or a solvate, **characterized in that** it is used to prepare a pharmaceutical composition for treating diseases, conditions, or symptoms related to KRas G12C activity or expression levels.

9. The use as described in claim 8, **characterized in that** the disease, symptom, or condition is selected from the group consisting of: pancreatic cancer, non-small cell lung cancer, small cell lung cancer, lung adenocarcinoma, lung squamous cell carcinoma, colon cancer, colorectal cancer, thyroid cancer, embryonal rhabdomyosarcoma, granulosa cell tumor of the skin, melanoma, liver cancer, rectal cancer, bladder cancer, pharyngeal cancer, breast cancer, prostate cancer, glioma, ovarian cancer, head and neck squamous cell carcinoma, cervical cancer, esophageal cancer, kidney cancer, skin cancer, lymphoma, gastric cancer, acute myeloid leukemia, myelofibrosis, B-cell lymphoma, monocytic leukemia, splenomegaly with polycythemia, eosinophilic leukocytosis, multiple myeloma, and various solid tumors and hematologic malignancies.
